# EUROPÄISCHE PATENTSCHRIFT

(11) **EP 2 413 799 B1**
(45) Veröffentlichungstag und Bekanntmachung des Hinweises auf die Patenterteilung: **10.07.2013**
(21) Anmeldenummer: 10711367.2
(22) Anmeldetag: 17.03.2010
(51) Int. Cl.: A61B 5/151

(54) **BLUTLANZETTENVORRICHTUNG MIT STECHTIEFENEINSTELLUNG**
BLOOD LANCET DEVICE INCLUDING ADJUSTMENT OF THE PRICKING DEPTH
DISPOSITIF À LANCETTE À RÉGLAGE DE LA PROFONDEUR D'UNE PIQÛRE

(30) Priorität: 03.04.2009 DE 102009016126; 18.06.2009 DE 102009025443
(43) Veröffentlichungstag der Anmeldung: 08.02.2012
(73) Patentinhaber: Gerresheimer Regensburg GmbH, 93047 Regensburg (DE)
(72) Erfinder: KNIE, Andreas, 93051 Regensburg (DE); BUTZ, Marion, 93049 Regensburg (DE)
(74) Vertreter: Hannke, Christian
(86) Internationale Anmeldenummer: PCT/EP2010/053426
(87) Internationale Veröffentlichungsnummer: WO 2010/112335

(56) Entgegenhaltungen:
- EP-A1- 1 810 615
- WO-A1-2005/077275
- WO-A2-2007/047812
- US-A1- 2002 029 059

## Beschreibung

Die Erfindung betrifft eine Blutlanzettenvorrichtung mit einer Stechtiefeneinstellung gemäß dem Oberbegriff des Patentanspruches 1. Bei Blutlanzettenvorrichtungen beziehungsweise Stechhilfen ist bekannt, dass zur Einstellung einer Stechtiefe verstellbare Anschläge im vorderen Bereich der Blutlanzettenvorrichtung verwendet werden, wogegen ein Lanzettenhalter, welcher die Lanzette umfasst, bei einem vorzugsweise federbeaufschlagten Stechvorgang, bei dem die Lanzette aus einem Gehäuse der Stechhilfe gefahren wird, anschlägt, wobei dieser Anschlag veränderbar ist, so dass die Lanzette unterschiedlich weit - je nach Einstellung des Anschlags - aus dem Gehäuse der Stechhilfe herausfährt. Die Konstruktionen derartig aufgebauter Stechhilfen mit integriertem veränderbaren Anschlag sind jedoch häufig komplex und erfordern eine Fixierung des Anschlages beziehungsweise des Anschlagelementes in einer gewünschten zu verändernden Position.

Ebenso ist gemäß der Druckschrift EP 1810615 A1 ein8e Blutlanzettenvorrichtung bekannt, bei welcher auf einem stiftartig ausgebildeten Gehäuse vorderseitig ein Kappenelement aufschraubbar ist, wobei dieses Kappenelement mittels eines Gewindes verdreht werden kann, um eine an dem Kappenelement vorderseitig angeordnete Auslassöffnung mehr oder weniger stark von dem restlichen Gehäuse zu beabstanden.

Ein derartiges an dem Gehäuse integral angeordnetes Kappenelement, welches sowohl die Auslassöffnung als auch den darin teilweise angeordneten Lanzettenhalter vollständig umfasst, ist insbesondere bei einer Bedienung der Blutlanzettenvorrichtung mittels nur einer Hand nur schwer in einer Position gegenüber dem restlichen Gehäuse einstellbar. Durch eine Veränderung des Abstandes der Auslassöffnung gegenüber dem restlichen Gehäuse wird ein unterschiedlich starker Austritt der Lanzettenspitze aus dem mit Ausnahme der Auslassöffnung vollständig geschlossenen Kappenelement ermöglicht.

WO 2007/047812 A2 zeigt eine Blutlanzettenvorrichtung mit einem Grundkörper und einer Lanzette, die ein- und ausfahrbar ist. Zumindest Teile dieser Lanzette sind mit antimikrobakteriellen Eigenschaften versehen. Die Lanzette tritt hierbei im Bereich einer Kappe aus.

US 2002/0029059 Au zeigt ebenso eine Blutlanzettenvorrichtung mit einer Kappe, die in ihrer Position verstellbar ist.

Es soll demzufolge eine Einrichtung für eine Stechtiefeneinstellung einer Blutlanzettenvorrichtung zur Verfügung gestellt werden, die eine einfache Handhabung in ihrer Verstellbarkeit auch mittels nur einer Hand und einer Absicherung gegen ungewolltes Verstellen der ausgewählten Stechtiefe ermöglicht.

Erfindungsgemäß erfolgt die Lösung der Aufgabe durch eine Blutlanzettenvorrichtung nach dem Gegenstand des Anspruches 1. Eine erfindungsgemäße Blutlanzettenvorrichtung weist einen Grundkörper zum Anordnen von Vorrichtungskomponenten auf. Ferner umfasst die Blutlanzettenvorrichtung eine Lanzettenaufnahmeeinrichtung zum Halten mindestens eines Lanzettenelementes, die zumindest abschnittsweise von dem Grundkörper umgeben ist, d.h. beispielsweise, dass sich das Lanzettenelement komplett innerhalb des Grundkörpers befinden kann, teilweise aus dem Grundkörper vorstehen kann oder der Grundkörper Öffnungen aufweist, durch die das Lanzettenelement zum Vorschein kommt.

Weiterhin ist eine mit dem Grundkörper gekoppelte Kappe zum Anordnen eines Stellrades auf einem zumindest teilweise zylinderförmigen Abschnitt der Kappe vorgesehen, um eine Mehrzahl an Austrittslängen des Lanzettenelements aus der Blutlanzettenvorrichtung einzustellen.

Eine Kappe kann hierbei als Fortsatz des Grundkörpers angesehen werden, die mit diesem einstückig beziehungsweise unlösbar oder lösbar z.B. durch Aufsetzen auf dem Grundkörper verbunden ist.

Das Stellrad weist einen Innengewindeabschnitt auf und der zylinderförmige Abschnitt ist mit einem Außengewindeabschnitt versehen, wobei die Gewindeabschnitte funktional derart miteinander verbunden sind, dass das Stellrad drehbar und in Längsrichtung des Grundkörpers verstellbar ist, d.h., dass mittels der Gewindeabschnitte die Drehbewegung des Stellrades derart mit einer Verstellung des Stellrades in Längsrichtung gekoppelt ist, dass durch eine Drehung des Stellrades zeitgleich eine Längsverstellung des Stellrades durchführbar ist.

Erfindungsgemäß ist das Stellrad derart von sich in Längsrichtung erstreckenden armartigen Segmenten umgeben, dass eine zur Einstellung der Austrittlänge erforderliche Betätigung des Stellrades lediglich in den segmentfreien Umfangsabschnitten durchführbar ist. Dies ist vorteilhaft, da durch die Segmente ein Anschlag vorgegeben ist, durch den die Finger- bzw. die Daumenbewegung begrenzt ist, wodurch ein kontrollierteres und ergonomisch vorteilhafteres Einstellen der Austrittslänge erfolgt.

In einer weiteren bevorzugten Ausführungsform der vorliegenden Erfindung weist die Kappe und/oder der Grundkörper einen Querschnitt auf, der in einer ersten Querschnittsrichtung der Breitenrichtung größer als der Durchmesser des Stellrades ist und in einer zweiten Richtung der Tiefenrichtung kleiner als der Querschnitt in der ersten Richtung ist. Dies ist vorteilhaft, da der Grundkörper und/oder die Kappe hierdurch eine Form aufweisen, aufgrund derer die Blutlanzettenvorrichtung definiert in der Hand liegt beziehungsweise auf intuitive Weise von dem Patienten in die Hand genommen wird und missverständliche Bedienungsschritte daher ausgeschlossen sind.

In einer weiteren bevorzugten Ausführungsform der vorliegenden Erfindung ist der zylinderförmige Abschnitt der Kappe zwischen den zwei den Abschnitt teilweise umschließenden armförmigen Segmenten der Kappe angeordnet. Diese Ausführungsform ist vorteilhaft, da durch jeweils ein Segment und das Stellrad ein Schlitz gebildet wird, der bevorzugt derart ausgestaltet ist, dass ein Eindringen eines Fingers beziehungsweise des Daumens in den Schlitz ausgeschlossen ist.

Der Grad der Umschließung des Stellrades durch die Segmente lässt ferner einen Rückschluss auf die in einem Betätigungsschritt maximal mögliche Drehung des Stellrades zu.

In einer weiteren bevorzugten Ausführungsform der vorliegenden Erfindung sind die Segmente gleichförmig ausgebildet. Dies ist vorteilhaft, da die Konstruktion und Herstellung der Kappe sowie Weiterentwicklungen wesentlich vereinfacht werden, da eine höhere Anzahl an Gleichteilen beziehungsweise gleichen Bereichen oder Abschnitten vorliegt.

In einer weiteren bevorzugten Ausführungsform der vorliegenden Erfindung sind sich die Segmente bezüglich des zylinderförmigen Abschnittes gegenüberliegend angeordnet. Dies ist vorteilhaft, da die Vorrichtung relativ symmetrisch beziehungsweise symmetrisch aufgebaut ist, wodurch die Montage der Kappe in verschiedenen Orientierungen erfolgen kann und diese dadurch beschleunigt und vereinfacht ist.

In einer weiteren bevorzugten Ausführungsform der vorliegenden Erfindung ist das Stellrad länger als die Segmente oder zumindest teilweise in Längsrichtung gegenüber den Segmenten vorstehend. Dies ist vorteilhaft, da auf diese Weise sicher gestellt ist, dass die Oberfläche des Stellrades, bevorzugt die ebene Oberfläche des Stellrades, die das Stellrad nach außen abschließt und die Austrittsöffnung für das Lanzettenelement aufweist, in gewünschter Weise an einer Behandlungsstelle beziehungsweise einem Körper angeordnet werden kann.

In einer weiteren bevorzugten Ausführungsform der vorliegenden Erfindung erstrecken sich eine erste Positionierungseinrichtung und eine weitere Positionierungseinrichtung zumindest teilweise in Längsrichtung, wobei eine der beiden Positionierungseinrichtungen an der Kappe und die andere Positionierungseinrichtung an dem Stellrad vorgesehen ist.

Die erste Positionierungseinrichtung besteht aus mindestens einem Zapfen und die weitere Positionierungseinrichtung ist wellenförmig, insbesondere zahnförmig ausgebildet. Der mindestens eine Zapfen ist zumindest zeitweise auslenkbar, d.h. biegbar. Das Verbiegen des Zapfens erfolgt derart, dass eine erste Fläche des Zapfens relativ gegenüber einer zweiten Fläche des Zapfens bewegt wird. Diese Ausführungsform ist vorteilhaft, da durch diese Anordnungen auf einfache und auf kostengünstige Weise ein Ratschenmechanismus vorsehbar ist, der ein schrittweises Einstellen der Austrittsweite des Lanzettenelementes aus der Blutlanzettenvorrichtung ermöglicht.

In einer weiteren bevorzugten Ausführungsform der vorliegenden Erfindung umgibt die erste Positionierungseinrichtung zumindest teilweise den zylinderförmigen Abschnitt beziehungsweise ist daran ausgebildet und stellt eine Außenverzahnung dar, deren Zähne Zahnflanken aufweisen, deren Mehrzahl im Wesentlichen gleich ausgebildet ist. Weiterhin ist zumindest ein Zapfen an dem Stellrad ausgebildet. Dies hat den Vorteil, dass die Verzahnung und der Zapfen in einem durch das Stellrad begrenzten Bereich geschützt zusammenwirken.

In einer weiteren bevorzugten Ausführungsform der vorliegenden Erfindung ist die erste Positionierungseinrichtung zumindest teilweise vom Stellrad umgeben. Das Stellrad weist zumindest teilweise eine Innenverzahnung auf und die Zähne weisen Zahnflanken auf, deren Mehrzahl im Wesentlichen gleich ausgebildet ist und die gegenüber der sich von einer Mittelachse des zylindrischen Körpers erstreckenden radialen Richtung geneigt sind. Ferner ist an dem zumindest teilweise zylinderförmigen Abschnitt ein Zapfen ausgebildet, besonders bevorzugt sind an dem Abschnitt zumindest zwei voneinander beabstandete Zapfen ausgebildet. Dies hat den Vorteil, dass das Zusammenwirken des Zapfens und der Verzahnung ebenfalls geschützt ausgeführt ist. Ferner kann je nach Produktionsverfahren die erste oder die weitere Positionierungseinrichtung an dem zylinderförmigen Abschnitt oder dem Stellrad angeordnet sein.

In einer weiteren bevorzugten Ausführungsform der vorliegenden Erfindung wäre es ebenfalls denkbar, die Außenverzahnung beziehungsweise den Zapfen an der Umfangsfläche des Stellrades vorzusehen und entsprechend die Innenverzahnung beziehungsweise einen Zapfen an mindesten einem der Segmente vorzusehen. Bevorzugt ist hierbei, dass die jeweilige Verzahnung mit den Zapfen zusammenwirkt.

In einer weiteren bevorzugten Ausführungsform der vorliegenden Erfindung weist zumindest ein Zahn der weiteren Positionierungseinrichtung eine Flanke auf, die im Vergleich zu der Mehrzahl der Flanken steiler ausgebildet ist. Steiler bezieht sich hierbei auf die Ausrichtung der Flanke gegenüber dem Punkt an dem die Flanke mit der Basis beziehungsweise dem Stellrad oder dem zylinderförmigen Abschnitt in Kontakt steht.

Bevorzugt ist die Flanke im Wesentlichen in einem Winkel von 90° zu der Basis ausgerichtet, wodurch diese Flanke die Drehbewegung des Stellrades beschränkt. Dies ist vorteilhaft da, eine Verstellung des Stellrades lediglich in einem vorgesehen Bereich möglich ist und so beispielsweise das Abdrehen des Stellrades verhindert wird.

In einer weiteren bevorzugten Ausführungsform der vorliegenden Erfindung sind an der Kappe und dem Stellrad zumindest teilweise Markierungen zur Bestimmung der Austrittslänge des Lanzettenelements aus der Blutlanzettenvorrichtung heraus vorgesehen. Dies ist vorteilhaft, da an Hand der Markierungen eine gewünschte Lanzettenaustrittslänge einfach einstellbar bzw. kontrollierbar ist.

Weitere Vorteile und Ausführungsformen ergeben sich aus den beigefügten Zeichnungen:

Darin zeigen:
- Fig. 1: Eine Vorderansicht einer erfindungsgemäßen Blutlanzettenvorrichtung;
- Fig. 2: eine Rückansicht einer erfindungsgemäßen Blutlanzettenvorrichtung;
- Fig. 3: eine Draufsicht auf die erfindungsgemäße Kappe und das erfindungsgemäße Stellrad in einem ersten Zustand;
- Fig. 4: eine Draufsicht auf die erfindungsgemäße Kappe und das erfindungsgemäße Stellrad in einem zweiten Zustand;
- Fig. 5: eine perspektivische Darstellung der erfindungsgemäßen Kappe und des er findungsgemäßen Drehrades;
- Fig. 6: eine erste Schnittdarstellung eines erfindungsgemäßen Stellrades;
- Fig. 7: eine zweite Schnittdarstellung eines erfindungsgemäßen Stellrades;
- Fig. 8: eine perspektivische Schnittdarstellung eines erfindungsgemäßen Stellrades;
- Fig. 9: eine perspektivische Darstellung einer erfindungsgemäßen Kappe;
- Fig. 10: eine perspektivische Darstellung eines erfindungsgemäßen Stellrades;
- Fig. 11a: eine Draufsicht auf eine weitere erfindungsgemäße Kappe;
- Fig. 11b: eine Vorderansicht der weiteren erfindungsgemäßen Kappe;
- Fig. 12a: eine Rückansicht der weiteren erfindungsgemäßen Kappe;
- Fig. 12b: eine perspektivische Ansicht der erfindungsgemäßen Kappe;
- Fig. 13a: eine Schnittdarstellung eines weiteren erfindungsgemäßen Stellrades;
- Fig. 13b: eine Schnittdarstellung eines mit der erfindungsgemäßen Kappe gekoppelten erfindungsgemäßen Stellrades;
- Fig. 14a: eine Schnittdarstellung der erfindungsgemäßen Kappe und des erfindungsgemäßen Stellrades in einem ersten Zustand;
- Fig. 14b: eine Schnittdarstellung der erfindungsgemäßen Kappe und des erfindungsgemäßen Stellrades in einem zweiten Zustand;
- Fig. 15a: eine dreidimensionale Schnittdarstellung eines erfindungsgemäßen Stellrades;
- Fig. 15b: eine dreidimensionale Schnittdarstellung eines erfindungsgemäßen Stellrades und einer erfindungsgemäßen Kappe in einem gekoppelten Zustand;
- Fig. 16: eine dreidimensionale Darstellung der weiteren erfindungsgemäßen Kappe.

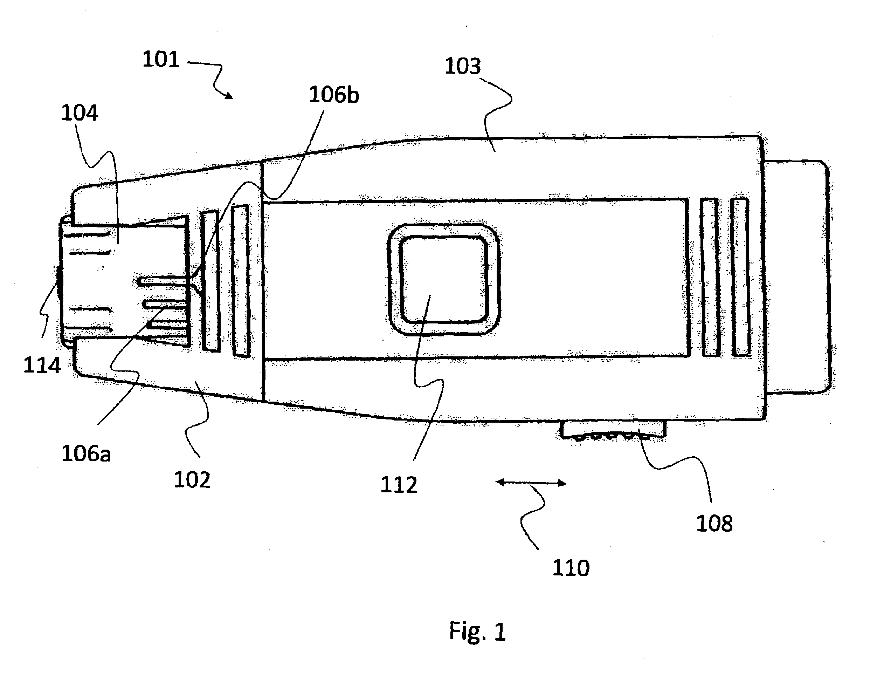

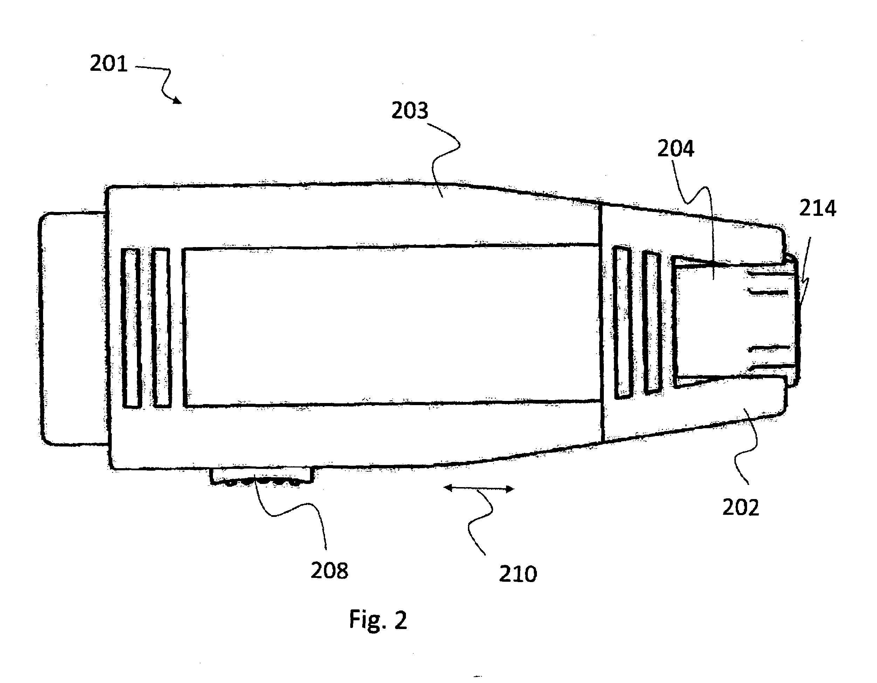

In den Fig. 1 und Fig. 2 ist eine erfindungsgemäße Blutlanzettenvorrichtung 101, 201 dargestellt. Die Blutlanzettenvorrichtung 101, 201 ist in der Fig. 1 von ihrer Vorderseite und in Fig. 2 von ihrer Rückseite aus wiedergegeben. Die Blutlanzettenvorrichtung 101, 201 weist eine Kappe 102, 202 auf, die mit einem Gehäuse 103, 203 gekoppelt ist. Ferner ist an der Kappe 102, 202 ein Stellrad 104, 204 angeordnet, das relativ beweglich gegenüber der Kappe 104, 204 ist und Markierungen 106a, 206a aufweist. Korrespondierend zu den Markierungen 106a, 206a des Stellrades 104, 204 weist die Kappe 102, 202 ebenfalls mindestens eine Markierung 106b, 206b auf. Aufgrund der Markierungen 106a, 206a und der mindestens einen Markierung 106b, 206b kann eine Bedienperson bzw. ein Patient eine Einstellung der Einstechtiefe nach Wunsch vornehmen bzw. die jeweils gegebene Konfiguration direkt ablesen.

Es ergibt sich daraus, dass die Bedienperson eine Auswahl einer gewünschten Einstechtiefe aus bevorzugt verschiedenen Einstechtiefen bzw. Austrittslängen (L1, L2, L3) durch ein Verdrehen des Stellrades 104, 204 vornehmen kann. Die Austrittslängen (L1, L2, L3) können hierbei in beliebiger Staffelung ausgeführt sein. Weiterhin ist denkbar, dass je nach Bedarf eine beliebige Anzahl an Austrittslängen vorgesehen sein kann.

Seitlich an der Blutlanzettenvorrichtung 101, 201 ist ein Handbestätigungselement 108, 208 zum Betätigen eines Schiebelementes, welches in diesen Darstellungen nicht näher wiedergegeben ist, angeordnet. Dieser Schieber kann entlang des Doppelpfeiles 110, 210 in Längsrichtung L der Blutlanzettenvorrichtung 101, 201 verschoben werden. Ein Druckknopf 112, 212 dient zum Vorspannen eines Lanzettenhalters und einer Lanzette innerhalb des Gehäuses 103, 203, um anschließend mittels des Druckknopfes 112, den eigentlichen Auslösevorgang durchzuführen, bei dem die Lanzette aus einer Austrittsöffnung 114, 214 kurzzeitig ausgefahren und vorzugsweise anschließend wieder eingefahren wird.

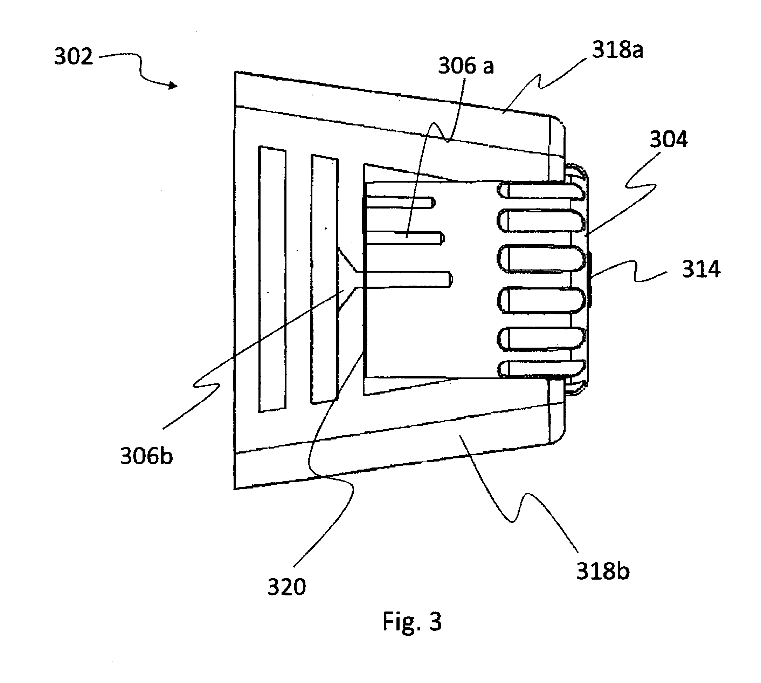

Unter Bezugnahme auf die Stechtiefeneinstellung beziehungsweise Stechtiefenverstellung ist in den Figuren 3, 4 und 5 eine Kappe 302, 402, 502 mit einem dazugehörigen Stellrad 304, 404, 504 wiedergegeben, wobei die Kappe 302, 402, 502 mit dem Stellrad, an der Vorderseite des Gehäuses der Blutlanzettenvorrichtung, angeordnet ist. Dies ist aus den Fig. 1 und Fig. 2 ersichtlich.

Die Kappe 302, 402, 502 zeichnet sich dadurch aus, dass sie das Stellrad 304, 404, 504 nicht vollständig umfasst, sondern sich lediglich seitlich nach vorne erstreckt. Hierdurch ist vorteilhaft möglich, dass das Stellrad 304, 404, 504 in Richtung des Doppelpfeils 316, 416, 516 verdreht werden kann, ohne dass eine ungewollte Verstellung des Stellrades 304, 404, 504 erfolgt, da ein seitliches Umfassen der Kappe 302, 402, 502 durch die Segmente 318a, 418a, 518a und 318b, 418b, 518b erfolgte.

In Fig. 3 befindet sich das Stellrad 304 in einer derartigen Drehstellung, dass eine größte Einstechtiefe einer hier nicht dargestellten Lanzette, welche aus der Austrittsöffnung 314 hervortritt, vorliegt. In dieser Position ist das Stellrad 304 gegenüber der Kappe 302 maximal eingefahren beziehungsweise derart verdreht, dass es in seinem rückwärtigen Bereich 320 den geringsten Abstand zu der Kappe 302 aufweist.

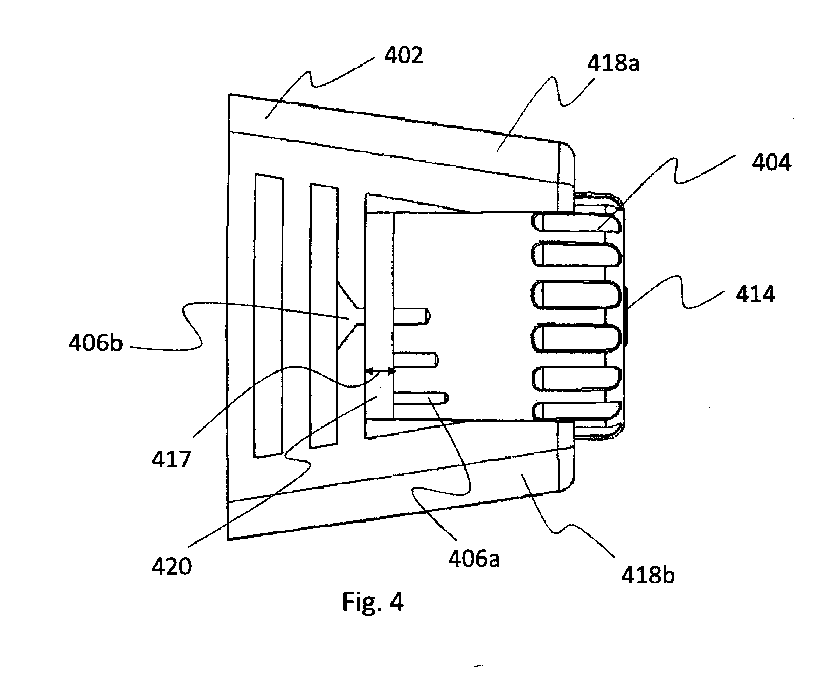

In Fig. 4 hingegen ist das Stellrad 404 derart verdreht, dass es eine geringste Einstechtiefe der aus der Austrittsöffnung 414 hervorstehenden Lanzette sicherstellt. In dieser Position ist das Stellrad 404 gegenüber der Kappe 402 in seinem rückwärtige Bereich 420 beabstandet. Wie es aus dem Bezugszeichen 417, welches den Abstand wiedergeben soll, hervorgeht.

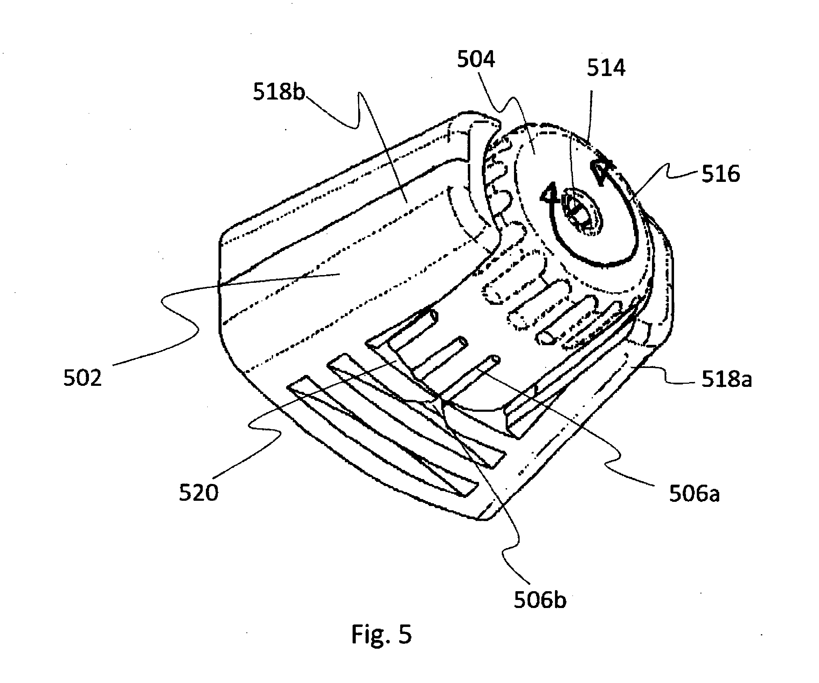

Fig. 5 weist eine perspektivische Darstellung der in Fig. 3 gezeigten Anordnung. Die Kappe 502 ist hierbei in Richtung des Doppelpfeils 520 bewegbar.

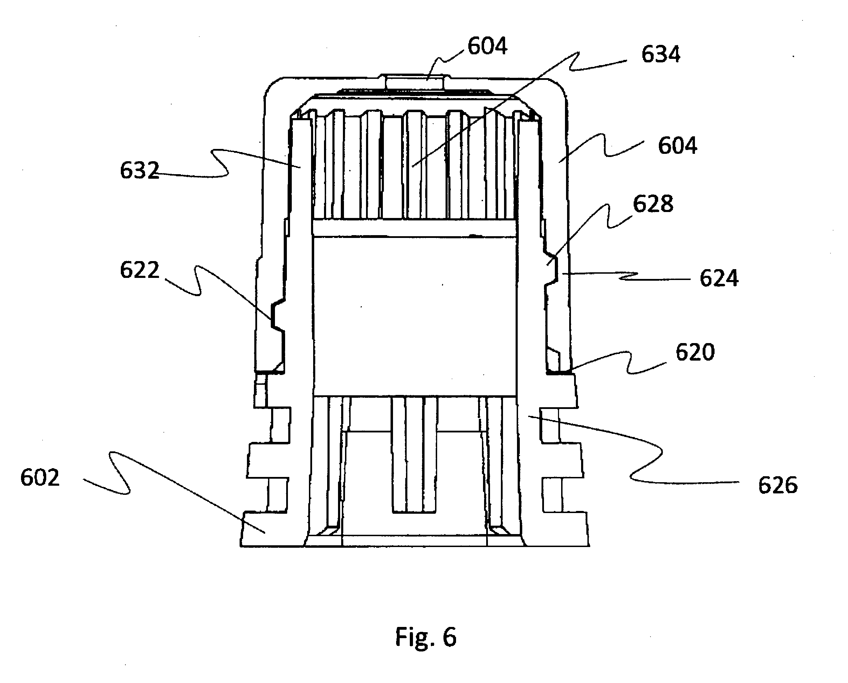

Auch in den Fig. 6 - 8 ist die Kappe zur Stechtiefeneinstellung beziehungsweise Stechtiefenverstellung zusammen mit dem Stellrad 604, 704, 804 wiedergegeben. Hierbei handelt es sich um Querschnittsdarstellungen.

Weiterhin ist den Fig. 6 - 8 zu entnehmen, dass das Stellrad 604, 704, 804 gegenüber der Kappe 602, 702, 802 mittels einer schematisch dargestellten Gewindeanordnung 622, 722, 822 verdreht werden kann, wobei das Stellrad 604, 704, 804 einen Innengewindeabschnitt 624, 724, 824 und der zylinderförmige Abschnitt 626, 726, 826 der Kappe 602, 702, 802 einen Außengewindeabschnitt 628, 728, 828 aufweist, so dass in Fig. 6 eine Drehstellung vorhanden ist, welche die höchste Einstechtiefe der Lanzette sicherstellt.

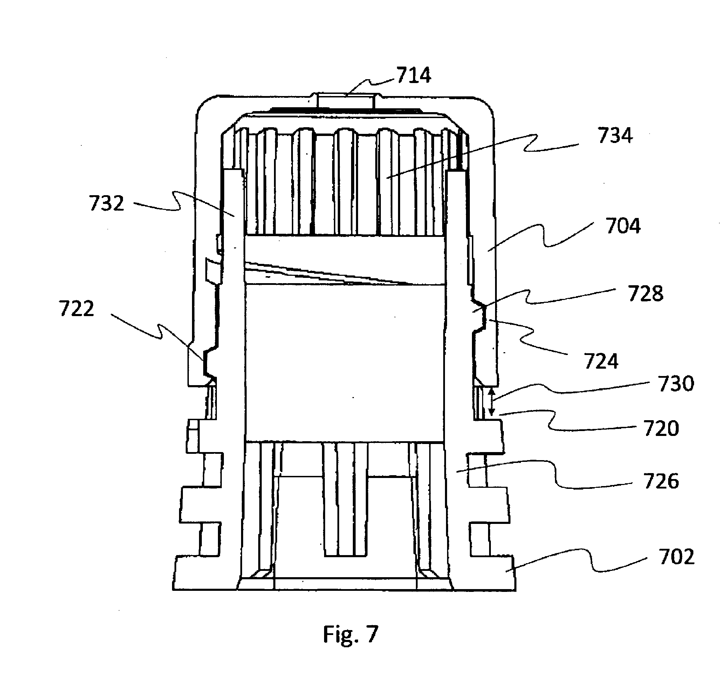

In Fig. 7 ist eine Drehstellung des Stellrades 704 wiedergegeben, die eine geringste Einstichtiefe wiedergibt. Im Zustand der geringsten Einstechtiefe ist ein Abstand 730 zwischen dem Stellrad 704 und der Kappe 702 vorhanden.

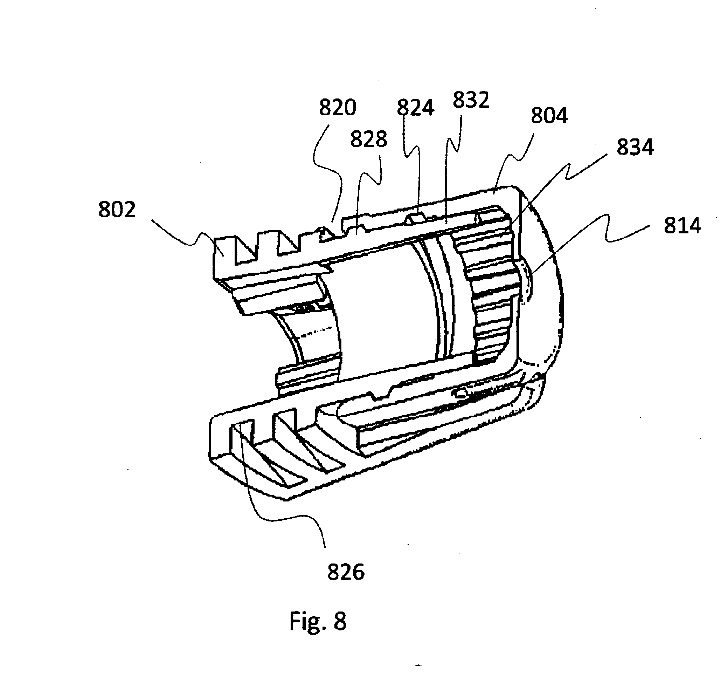

In Fig. 8 ist eine perspektivische Darstellung der in Fig. 7 gezeigten Anordnung dargestellt.

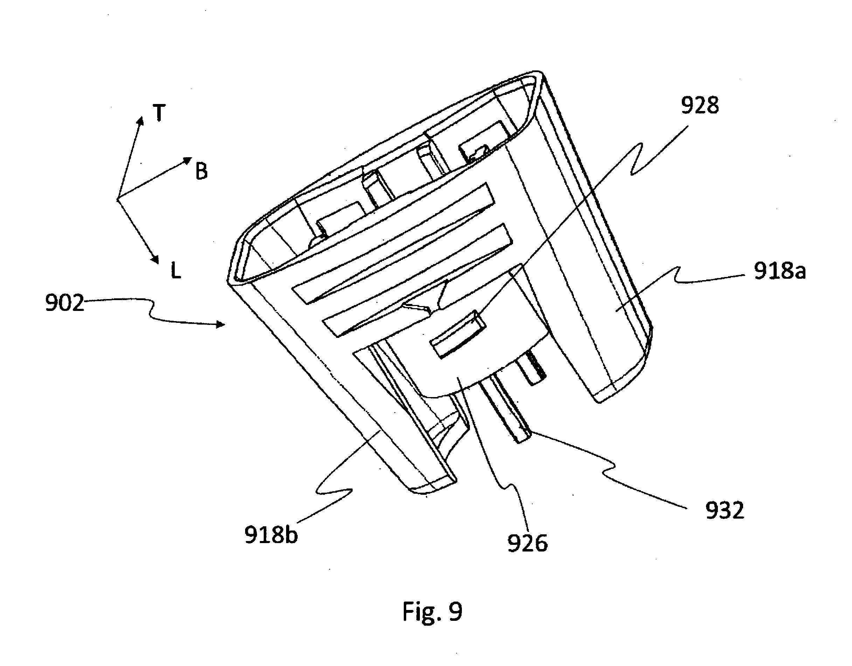

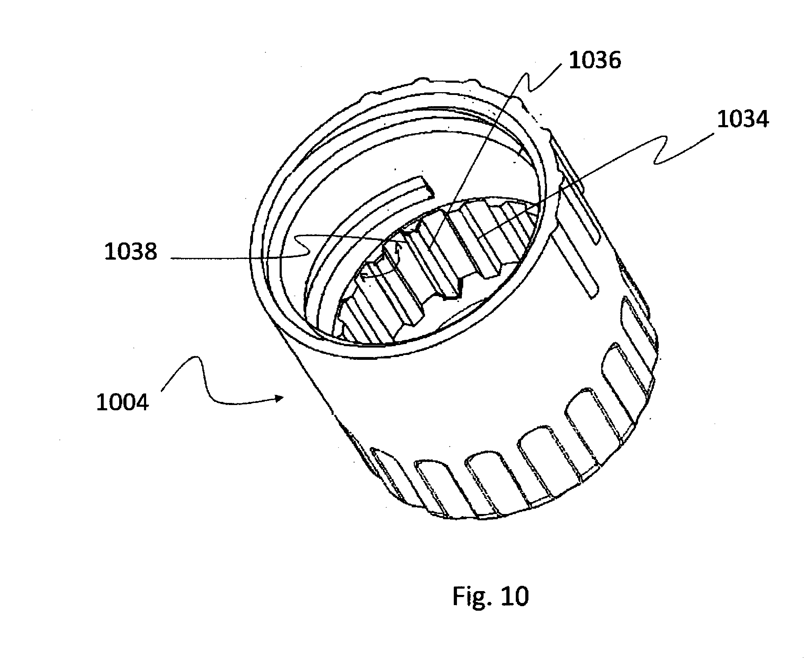

In den Fig. 9 und Fig. 10 ist die Kappe 902 bzw. das Stellrad 1004 mit daran integrierten Zapfen 932, dargestellt, welche die Aufgabe haben, in eine verzahnungsartige innenseitig liegende Ausbildung, 1034 an der Kappe 902 oder an dem Stellrad 1004 einzugreifen. Dies soll eine Art "Ratscheneffekt" ermöglichen, um eine stufenweise Verstellung des Stellrades 1004 gegenüber der Kappe 902 sicherzustellen. Der Pfeil B repräsentiert die Breitenrichtung bzw. die erste Querschnittsrichtung der Kappe und der Pfeil T repräsentiert die Tiefenrichtung bzw. die zweite Querschnittsrichtung der Kappe.

Ebenso ist ein Zahn, 1036 dieser ratschenartigen Ausbildung 934, 1034 derart ausgebildet, dass diese einseitig oder beidseitig eine höhere Flanke 938, 1038 aufweist. Hierdurch wird eine Art Anschlag erhalten, so dass das Stellrad 1004 nur bis zu einer bestimmten Maximaldrehung verdreht werden kann. Dies stellt sicher, dass das Stellrad 1004 nicht unerwünscht abzudrehen ist.

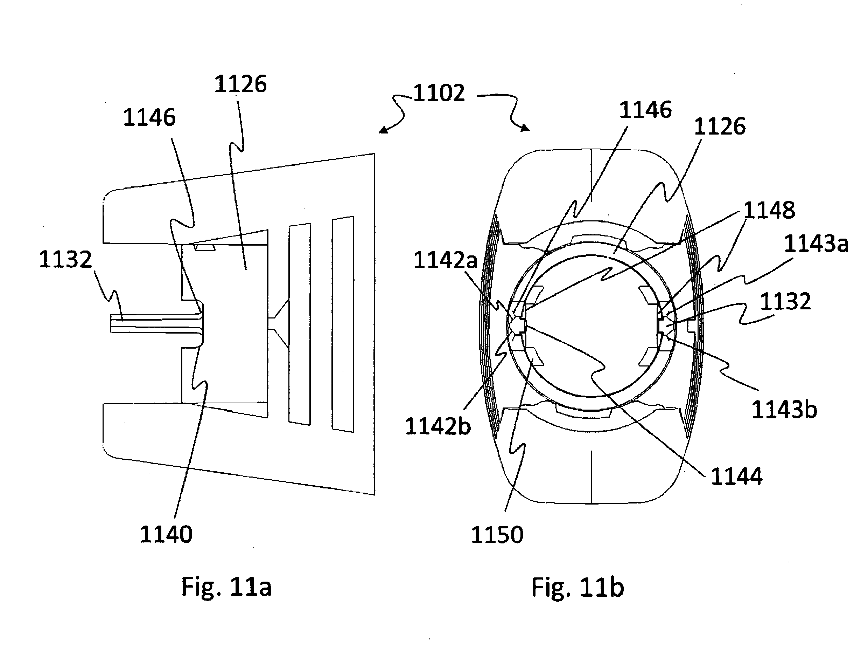

In den Figuren 11a und 11 b ist eine weitere Ausführungsform der Kappe 1102 dargestellt, in der die Zapfen 1132 in einer Ausnehmung 1140 des zylinderförmigen Abschnitts 1126 angeordnet sind. Der Zapfenfuß 1146 bzw. der Übergang vom Zapfen 1132 zu dem zylinderförmigen Abschnitt 1126 ist hierbei bevorzugt kurvenförmig ausgebildet, dies hat den Vorteil, dass durch das Auslenken eines der Zapfen 1132 keine bzw. eine wesentlich reduzierte Kerbwirkung in der Verbindungsstelle zwischen dem Zapfen 1132 und dem zylinderförmigen Abschnitt 1126 besteht, dies führt zu einer höheren Belastbarkeit des Zapfens 1132 bzw. zu einer Lebensdauersteigerung der Kappe.

Wie aus der Figur 11b hervorgeht, weisen die Zapfen 1132 Flanken 1142a und 1142b auf, die gegenüber dem Zapfenrücken 1144 geneigt sind. An den Flanken 1142a und 1142b können die Zähne der ratschenartigen Ausbildung des Stellrades (nicht dargestellt) abgleiten. Die Seitenbereiche 1143a und 1143b der Zapfen 1132 können beispielsweise mit einem Zahn (nicht gezeigt) zum Blockieren der Drehbewegung eines Stellrades dienen. Hierbei ist jedoch auch denkbar, dass der Zahn zum Blockieren der Drehbewegung mit einem der Seitenbereichen 1143a und 1143b und/oder mit einer der Flanken 1142a und 1142b zusammenwirkt bzw. in Kontakt kommt.

Es ist ebenfalls vorstellbar, dass zeitgleich zwei Zähne mit zwei Zapfen 1132 zusammenwirken. Der Zahnrücken ≤ kann sich in Längsrichtung L ebenfalls zumindest teilweise über den zylinderförmigen Abschnitt 1126 erstrecken und kann mit einer Zapfenbasis 1148 verbunden sein bzw. zusammenwirken, wodurch die Stabilität des Zapfens 1132 und der gesamten Kappe 1102 erhöht wird.

Die stabförmigen Elemente 1150 können ebenfalls zur Stabilisierung der Kappe 1102 beitragen. Ferner ist denkbar, dass diese Elemente 1150 für die Kopplung der Kappe 1102 mit einem Gehäuse oder Grundkörper dienen bzw. eine Führung für ein Lanzettenelement bzw. eine Lanzettenelementaufnahme bilden.

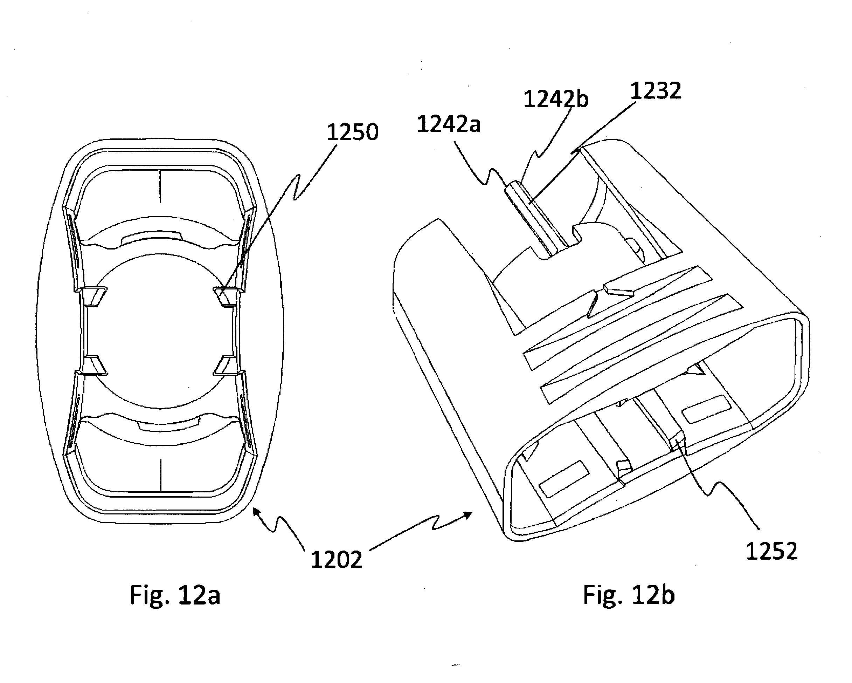

In den Figuren 12a und 12b ist ebenfalls eine Kappe 1202 gezeigt. Aus der perspektivischen Darstellung der Figur 12b lassen sich die Flanken 1242a und 1242b der Zapfen 1232 erkennen. Ferner ist aus den Darstellungen ersichtlich, dass die stabförmigen Elemente 1250 mit geneigten Flächen 1252 ausgeführt sein können, wobei eine solche Ausführung das Zusammenwirken der Kappe 1202 mit beispielsweise einem Gehäuse erleichtert.

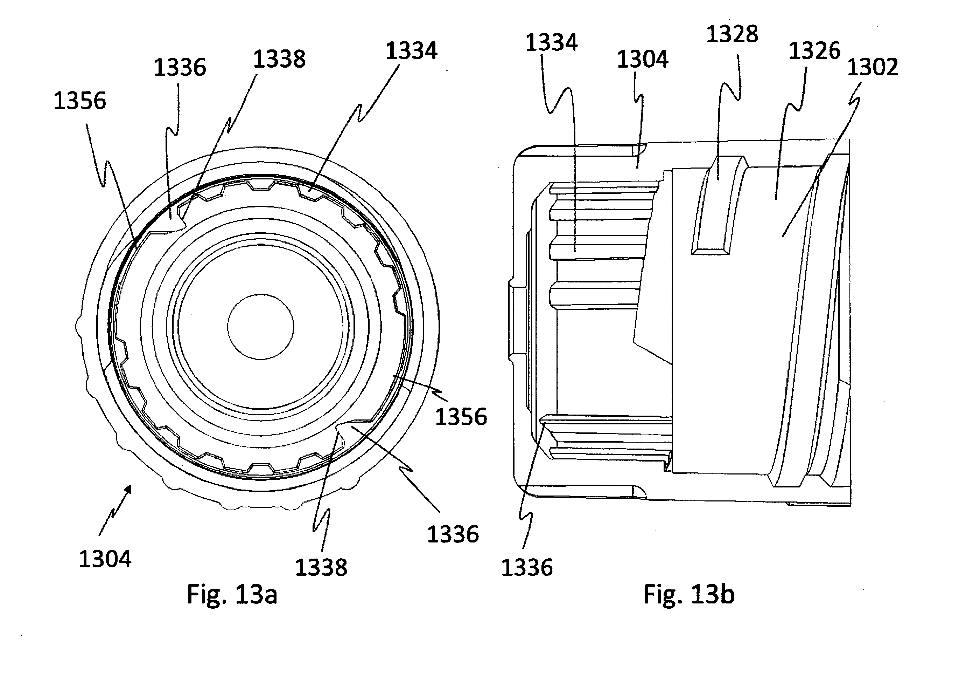

Die Figuren 13a und 13b zeigen ein Stellrad 1304, das eine ratschenartige Ausbildung 1334 aufweist, die bevorzugt aus einer Vielzahl gleich ausgebildeter Zähne besteht. Die ratschenartige Ausbildung 1334 kann sich hierbei über den gesamten Innenumfang des Stellrades 1304 erstrecken, wobei bevorzugt lediglich ein bzw. besonders bevorzugt zwei Abschnitte des Innenumfangs mit der ratschenartigen Ausbildung 1334 versehen sind und die Summe der Abschnitte einem Umfangsanteil entspricht, der bevorzugt kleiner ist als der Innenumfang des Stellrades 1304. Zwischen den ratschenartigen Ausbildungen 1334 sind daher bevorzugt gleichförmige und sich in Umfangsrichtung erstreckende Bereiche 1356 vorgesehen. Diese Ausführungsvariante hat den Vorteil, dass das Stellrad 1304 einfacher und schneller herstellbar ist, beispielsweise im Vergleich zu einer Ausführungsform, in der sich die ratschenartige Ausbildung 1334 über den gesamten Umfang erstreckt.

Ein Zahn 1336 einer oder bevorzugt beider ratschenartigen Ausbildungen 1334 weist eine höhere und/oder steilere Flanke 1338 auf. Durch das Zusammenwirken der Flanke 1338 mit einem Zapfen der Kappe 1302 wird ein Abgleiten des Zapfens verhindert, wodurch der elastische Zapfen nicht Auslenkbar ist, d.h. eine Drehbewegung des Stellrades 1304 blockiert wird.

Ferner ist in Figur 13b ein Außengewindeabschnitt 1328 der Kappe 1302 gezeigt, der sich in dieser Darstellung in einteiliger Form um den zylinderförmigen Abschnitt 1326 erstreckt.

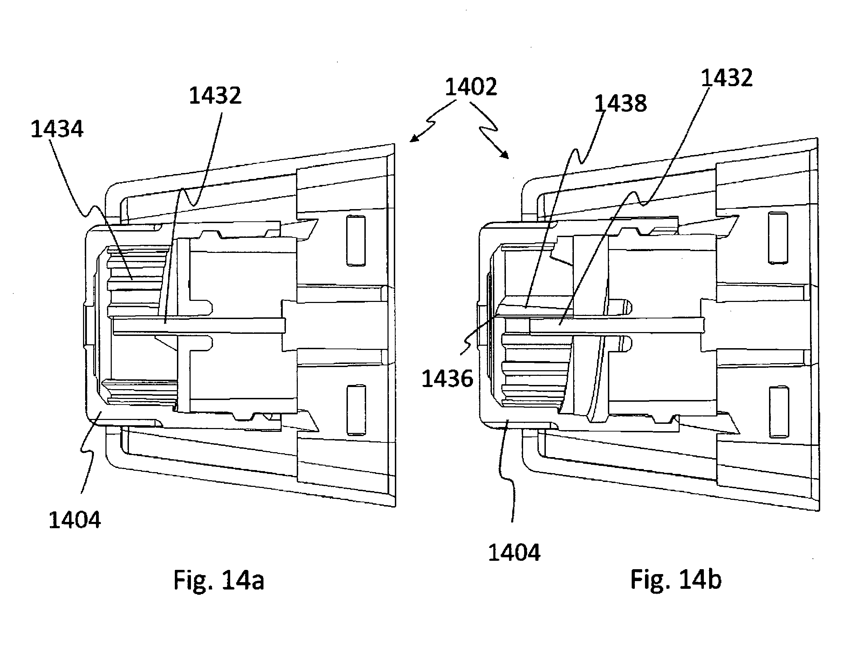

In den Figuren 14a und 14b sind zwei Zustände einer gekoppelten Stellrad-Kappen-Anordnung dargestellt. In Figur 14a ist ein Zustand gezeigt, in dem ein Lanzettenelement gegenüber dem in Figur 14b gezeigten Zustand, weiter aus dem Stellrad 1404 heraus austreten kann.

Es ist aus beiden Darstellungen (14a und 14b) ersichtlich, dass die ratschenartige Ausbildung 1434 Zähne aufweist, die unterschiedliche Längen aufweisen. Die einzelnen Zähne der ratschenartigen Ausbildung 1434 sind in ihrer Länge derart ausgebildet, dass bei einem Verdrehen des Stellrades 1404 und dem daraus resultierenden Versatz des Stellrades 1404 in Längsrichtung L stets vergleichbare Flächenanteile der ratschenartigen Ausbildung 1434 mit dem Zapfen 1432 in Kontakt stehen. Bevorzugt sind die Flächenanteile stets gleich groß, da aufgrund einer solchen Ausgestaltung die zum Drehen des Stellrades 1404 erforderliche Kraft stets gleich ist bzw. stets der gleiche Kraftverlauf vorliegt. Dies bedeutet, dass bevorzugt lediglich beim Zusammenwirken der Zapfen 1432 mit einer Flanke 1438 des Zahns 1436 ein veränderter Kraftverlauf vorliegt.

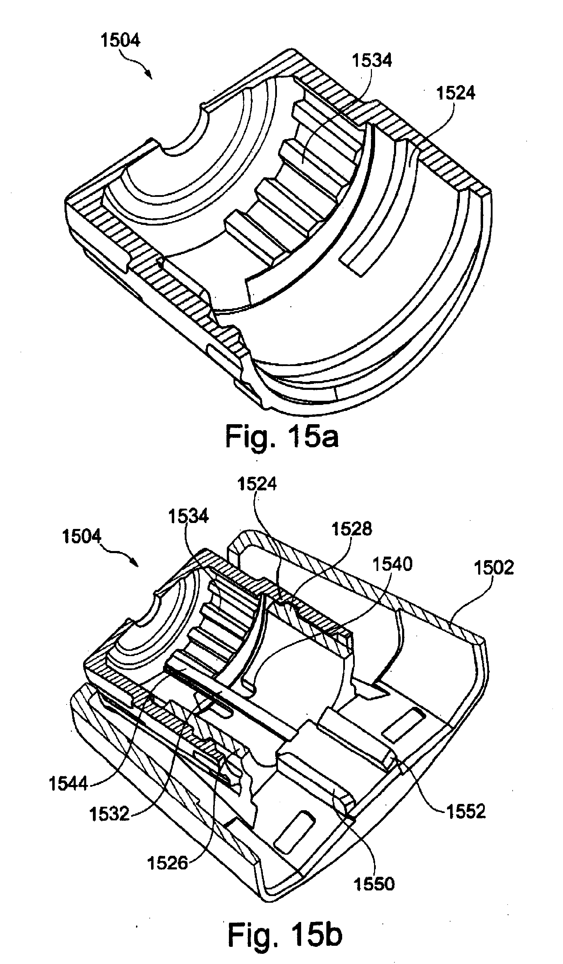

Aus den Figuren 15a und 15b gehen dreidimensionale Schnittdarstellungen des Stellrades 1504 und der Kappe 1502 hervor. Es ist ersichtlich, dass die ratschenartige Ausbildung 1534 Zähne umfasst, die unterschiedliche Längen aufweisen, bzw. die Länge der Zähne in Abhängigkeit von der jeweils in Umfangsrichtung vorgesehen Position verschieden ist. Bevorzugt besteht zwischen der Länge der Zähne und der jeweiligen Position in Umfangsrichtung ein lineares Verhältnis. Besonders bevorzugt ist die Länge der einzelnen Zähne von der Steigung des Innengewindes 1524 bzw. des Außengewindes 1528 abhängig. Ferner ist denkbar, dass durch die unterschiedlich langen Zähne eine Kurve gebildet wird, die parallel zu dem Gewinde verläuft.

Weiterhin ist aus Fig. 15b ersichtlich, dass sich der Zapfenrücken 1544 auf dem Zapfen 1532 und dem zylinderförmigen Abschnitt 1526 erstreckt. Der Zapfen 1532 ist hierbei in einer Ausnehmung 1540 des zylinderförmigen Abschnitts 1526 angeordnet. Die stabförmigen Elemente 1550 weisen in dieser Darstellung (Fig. 15b) geneigte Flächen 1552 auf, durch die ein Zusammenwirken der Kappe 1502 mit weiteren Einrichtungen erleichtert bzw. ermöglicht wird.

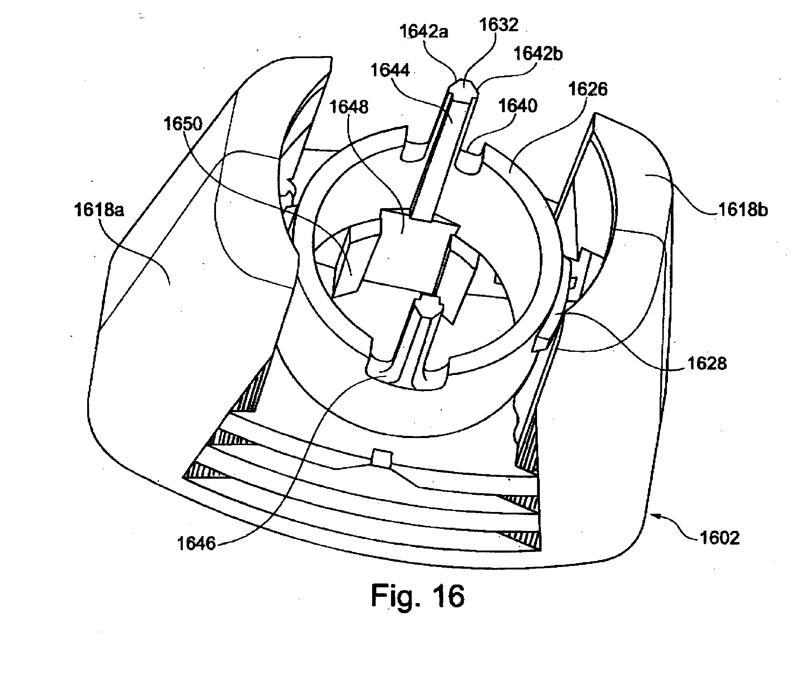

In Figur 16 ist in einer dreidimensionalen Darstellung eine Kappe 1602 gezeigt. Diese Kappe 1602weist zwei Segmente 1618a und 1618b auf, zwischen denen ein zylinderförmiger Abschnitt 1626 angeordnet ist. An dem zylinderförmigen Abschnitt 1626 ist ein Gewindeabschnitt 1628 vorgesehen, der mit einem Innengewinde eines Stellrades zusammenwirken kann.

Zwei Zapfen 1632 sind jeweils unterschiedlichen Ausnehmung 1640 vorgesehen. Jeder Zapfen 1632 weist hierbei einen pfeilförmigen Querschnitt auf. Die Spitze des pfeilförmigen Querschnitts wird zumindest von zwei Flanken 1642a und 1642b gebildet, wobei die Enden der in Spitzenrichtung orientierten Flanken miteinander in Kontakt stehen können, bevorzugt jedoch an einem dem Zapfenrücken 1644 gegenüberliegenden Abschnitt enden. Dieser Abschnitt kann hierbei parallel zu dem Zapfenrücken 1644 ausgeführt sein. Die Zapfen 1632 können, wie in dieser Figur dargestellt, jeweils einen Zapfenfuß 1646 aufweisen, der einen kurvenförmigen Übergang vom Zapfen 1632 auf den zylinderförmigen Abschnitt 1626 bildet.

Der Zapfenrücken 1644 erstreckt sich über den gesamten Zapfen 1632 und zumindest abschnittsweise über den zylinderförmigen Abschnitt 1626 bis zu einer Zapfenbasis 1648, die zumindest abschnittsweise in den durch den zylinderförmigen Abschnitt 1626 gebildeten Raum hineinragt.

Sämtliche in den Anmeldungsunterlagen offenbarten Merkmale werden als erfindungswesentlich beansprucht, sofern sie einzeln oder in Kombination gegenüber dem Stand der Technik neu sind.

**Bezugszeichenliste**

| | |
|---|---|
| 101, 201 | Blutlanzettenvorrichtung |
| 102,202,302,402,502,602,702,802,902, | Kappe |
| 1102, 1202, 1302, 1402, 1502, 1602103, 203, | Gehäuse beziehungsweise Grundkörper |
| 104, 204, 304, 404, 504, 604, 704, 804, 1004 1304,1404,1504 | Stellrad |
| 106a, 306a, 406a, 506a | Markierungen |
| 106b, 306b, 406b, 506b | Markierungen |
| 108, 208 | Handbetätigungselement |
| 110, 210 | Doppelpfeil (Fig. 1 und Fig. 2) |
| 112 | Druckknopf |
| 114, 214, 314, 414, 514, 614, 714, 814 | Austrittsöffnung |
| 516 | Doppelpfeil (Fig. 3 bis 5) |
| 417 | Abstand |
| 318a, 418a, 518a, 918a, 1618a | Segment |
| 318b, 418b, 518b, 918b, 1618b | Segment |
| 320, 420, 520, 620, 720, 820 | rückwärtiger Bereich |
| 622, 722, 822 | Gewindeanordnung |
| 624, 724, 824, 1524 | Innengewindeabschnitt |
| 626, 726, 826, 926, 1126, 1326, 1526, 1626 | zylinderförmiger Abschnitt |
| 628, 728, 828, 928, 1328, 1528, 1628 | Außengewindeabschnitt |
| 730 | Abstand |
| 632, 732, 832, 932, 1132, 1232, 1432, 1532, 1632 | Zapfen |
| 1034, 1334, 1434, 1534 | ratschenartige Ausbildung |
| 1036, 1336, 1436 | Zahn |
| 1038, 1338, 1438 | höhere Flanke |
| 1140, 1540, 1640 | Ausnehmung |
| 1142a/b, 1242a/b, 1642a/b | Flanke des Zapfens |
| 1143a/b | Seitenbereiche des Zapfens |
| 1144, 1544, 1644 | Zapfenrücken |
| 1146, 1646 | Zapfenfuß |
| 1148, 1648 | Zapfenbasis |
| 1150, 1250, 1550, 1650 | stabförmige Elemente |
| 1252, 1552 | geneigte Fläche |
| 1356 | gleichförmiger Bereich |
| L | Längsrichtung |
| B | ersten Querschnittsrichtung bzw. Breitenrichtung |
| T | zweite Querschnittsrichtung bzw. Tiefenrichtung |

## Patentansprüche

1. Blutlanzettenvorrichtung mit einem Grundkörper (103, 203, 303, 403, 503) zum Anordnen von Vorrichtungskomponenten, einer Lanzettenaufnahmeeinrichtung zum Halten mindestens eines Lanzettenelementes, die zumindest abschnittsweise von dem Grundkörper (103, 203, 303, 403, 503) umgeben ist, und einer mit dem Grundkörper gekoppelten Kappe (102, 202, 302, 402, 502, 602, 702, 802, 902, 1102, 1202, 1302, 1402, 1502, 1602) zum Anordnen eines Stellrades (104, 204, 304, 404, 504, 604, 704, 804, 1004, 1304, 1404, 1504) auf einem zumindest teilweise zylinderförmigen Abschnitt (626, 726, 826) der Kappe, um eine Mehrzahl an Austrittslängen (L1, L2, L3) des Lanzettenelements aus der Blutlanzettenvorrichtung (101, 201, 301, 401, 501) einzustellen,
wobei das Stellrad derart von sich in Längsrichtung (L) erstreckenden armartigen Segmenten (318a, 418a, 518a, 1618a, 318b, 418b, 518b, 1618b) umgeben ist, dass eine zur Einstellung der Austrittslänge erforderliche Betätigung des Stellrades lediglich in den segmentfreien Umfangsabschnitten durchführbar ist und zwei Positioniereinrichtungen (932, 1032, 1132, 1232, 1432, 1532, 1632, 934, 1034, 1334, 1434, 1534) vorgesehen sind, wobei eine der beiden Positioniereinrichtungen an der Kappe und die weitere Positioniereinrichtung an dem Stellrad ausgebildet ist,
**dadurch gekennzeichnet,dass**
das Stellrad einen Innengewindeabschnitt (624, 724, 824, 1524) und der zylinderförmige Abschnitt (626, 726, 826) einen Außengewindeabschnitt (628, 728, 828, 1328, 1528, 1628) aufweist, wobei die Gewindeabschnitte funktional derart miteinander verbunden sind, dass das Stellrad drehbar und in Längsrichtung (L) des Grundkörpers verstellbar ist und
die weitere Positioniereinrichtung Zähne mit Zahnflanken aufweist und zumindest ein Zahn der weiteren Positioniereinrichtung eine Flanke aufweist, die im Vergleich zu der Mehrzahl an Flanken steiler ausgebildet ist.

2. Blutlanzettenvorrichtung nach Anspruch 1,
**dadurch gekennzeichnet, dass**
die Kappe (102, 202, 302, 402, 502, 602, 702, 802, 902, 1102, 1202, 1302, 1402, 1502, 1602) und/oder der Grundkörper (103, 203, 303, 403, 503) einen Querschnitt aufweist, der in einer ersten Querschnittsrichtung (B) größer als der Durchmesser des Stellrades (104, 204, 304, 404, 504, 604, 704, 804, 1004, 1304, 1404, 1504) und in einer zweiten Querschnittsrichtung (T) kleiner als der Querschnitt in der ersten Querschnittsrichtung (B) ist.

3. Blutlanzettenvorrichtung nach Anspruch 1 oder 2,
**dadurch gekennzeichnet,dass**
der zylinderförmige Abschnitt der Kappe (102, 202, 302, 402, 502, 602, 702, 802, 902, 1102, 1202, 1302, 1402, 1502, 1602) zwischen den zwei den Abschnitt (102, 202, 302, 402, 502, 602, 702, 802, 902, 1102, 1202, 1302, 1402, 1502, 1602) teilweise umschließenden armförmigen Segmenten (318a, 418a, 518a, 1618a, 318b, 418b, 518b, 1618b) der Kappe (102, 202, 302, 402, 502, 602, 702, 802, 902, 1102, 1202, 1302, 1402, 1502, 1602) angeordnet ist.

4. Blutlanzettenvorrichtung nach Anspruch 3,
**dadurch gekennzeichnet,dass**
die Segmente (318a, 418a, 518a, 1618a, 318b, 418b, 518b, 1618b) gleichförmig ausgebildet sind.

5. Blutlanzettenvorrichtung nach Anspruch 3 oder 4,
**dadurch gekennzeichnet,dass**
die Segmente bezüglich des zylinderförmigen Abschnittes sich gegenüberliegend angeordnet sind.

6. Blutlanzettenvorrichtung nach einem der Ansprüche 3 bis 5,
**dadurch gekennzeichnet,dass**
das Stellrad (104, 204, 304, 404, 504, 604, 704, 804, 1004, 1304, 1404, 1504) länger als die Segmente (318a, 418a, 518a, 1618a, 318b, 418b, 518b, 1618b) ist oder zumindest teilweise in Längsrichtung (L) gegenüber den Segmenten vorsteht.

7. Blutlanzettenvorrichtung nach Anspruch 6,
**dadurch gekennzeichnet,dass**
sich eine erste Positionierungseinrichtung (932, 1032, 1132, 1232, 1432, 1532, 1632) und eine weitere Positionierungseinrichtung (934, 1034, 1334, 1434, 1534) zumindest teilweise in Längsrichtung (L) erstrecken sowie die erste Positionierungseinrichtung (932, 1032, 1132, 1232, 1432, 1532, 1632) mindestens aus einem Zapfen (932, 1032, 1132, 1232, 1432, 1532, 1632) besteht und die weitere Positionierungseinrichtung (934, 1034, 1334, 1434, 1534) wellenförmig, insbesondere zahnförmig, ausgebildet ist, wobei der mindestens eine Zapfen (932, 1032, 1132, 1232, 1432, 1532, 1632) zumindest zeitweise auslenkbar ist.

8. Blutlanzettenvorrichtung nach Anspruch 7,
**dadurch gekennzeichnet,dass**
die weitere Positionierungseinrichtung (934, 1034, 1334, 1434, 1534) zumindest teilweise den zylindrischen Abschnitt (626, 726, 826) umgibt und eine Außenverzahnung (934, 1034, 1334, 1434, 1534) aufweist und die Zähne Zahnflanken aufweisen, deren Mehrzahl im Wesentlichen gleich ausgebildet ist.

9. Blutlanzettenvorrichtung nach Anspruch 8,
**dadurch gekennzeichnet,dass**
zumindest ein Zapfen (932, 1032, 1132, 1232, 1432, 1532, 1632) an dem Stellrad (104, 204, 304, 404, 504, 604, 704, 804, 1004, 1304, 1404, 1504) ausgebildet ist.

10. Blutlanzettenvorrichtung nach Anspruch 8,
**dadurch gekennzeichnet, dass**
die erste Positionierungseinrichtung (932, 1032, 1132, 1232, 1432, 1532, 1632) zumindest teilweise vom Stellrad (104, 204, 304, 404, 504, 604, 704, 804, 1004, 1304, 1404, 1504) umgeben ist, das Stellrad zumindest teilweise eine Innenverzahnung (934, 1034, 1334, 1434, 1534) aufweist und die Zähne Zahnflanken aufweisen, deren Mehrzahl im Wesentlichen gleich ausgebildet ist.

11. Blutlanzettenvorrichtung nach Anspruch 10,
**dadurch gekennzeichnet,dass**
an dem zumindest teilweise zylinderförmigen Abschnitt (626, 726, 826) ein Zapfen (932, 1032, 1132, 1232, 1432, 1532, 1632) ausgebildet ist, bevorzugt an dem Abschnitt zumindest zwei voneinander beabstandete Zapfen (932, 1032, 1132, 1232, 1432, 1532, 1632) ausgebildet sind.

12. Blutlanzettenvorrichtung nach 7 bis 11,
**dadurch gekennzeichnet, dass**
zumindest ein Zahn (936, 1036, 1336, 1436) der weiteren Positionierungseinrichtung (934, 1034, 1334, 1434, 1534) eine Flanke aufweist, die im Vergleich zu der Mehrzahl der Flanken steiler ausgebildet ist, wobei durch diese Flanke die Drehbewegung des Stellrades (104, 204, 304, 404, 504, 604, 704, 804, 1004, 1304, 1404, 1504) beschränkt ist.

13. Blutlanzettenvorrichtung nach einem der vorangegangenen Ansprüche,
**dadurch gekennzeichnet, dass**
die Kappe (102, 202, 302, 402, 502, 602, 702, 802, 902, 1102, 1202, 1302, 1402, 1502, 1602) und das Stellrad (104, 204, 304, 404, 504, 604, 704, 804, 1004, 1304, 1404, 1504) zumindest teilweise Markierungen (106a, 206a, 106b, 206b) zur Bestimmung der Austrittslänge des Lanzettenelements aufweisen.

## Claims

1. A blood lancet apparatus with a base member (103, 203, 303, 403, 503) for the arrangement of apparatus components, a lancet-receiving device for holding at least one lancet element, which is surrounded at least locally by the base member (103, 203, 303, 403, 503), and a cap (102, 202, 302, 402, 502, 602, 702, 802, 902, 1102, 1202, 1302, 1402, 1502, 1602) coupled to the base member for the arrangement of a setting wheel (104, 204, 304, 404, 504, 604, 704, 804, 1004, 1304, 1404, 1504) on a portion (626, 726, 826) of the cap which is cylindrical at least in part, in order to set a majority of exit lengths (L1, L2, L3) of the lancet element out of the blood lancet apparatus (101, 201, 301, 401, 501), wherein the setting wheel is surrounded by arm-like segments (318a, 418a, 518a, 1618a, 318b, 418b, 518b, 1618b) extending in the longitudinal direction (L), in such a way that an actuation of the setting wheel required for setting the exit length is capable of being carried out only in the segment-free peripheral portions and two positioning devices (932, 1032, 1132, 1232, 1432, 1532, 1632, 934, 1034, 1334, 1434, 1534)are provided, in which case one of the two positioning devices is provided on the cap and the other positioning device is provided on the setting wheel, **characterized in that**
the setting wheel has an internally threaded portion (624, 724, 824, 1524) and the cylindrical portion (626, 726, 826) has an externally threaded portion (628, 728, 828, 1328, 1528, 1628), wherein the threaded portions are connected to one another functionally in such a way that the setting wheel is rotatable and adjustable in the longitudinal direction (L) of the base member, and the further positioning device has teeth with tooth-flanks and at least one tooth of the further positioning device has a tooth-flank which is made steeper as compared with the majority of the flanks.

2. A blood lancet apparatus according to claim 1, **characterized in that** the cap (102, 202, 302, 402, 502, 602, 702, 802, 902, 1102, 1202, 1302, 1402, 1502, 1602) and/or the base member (103, 203, 303, 403, 503) has or have a cross-section which in a first cross-sectional direction (B) is greater than the diameter of the setting wheel (104, 204, 304, 404, 504, 604, 704, 804, 1004, 1304, 1404, 1504) and in a second cross-sectional direction (T) is smaller than the cross-section in the first cross-sectional direction (B).

3. A blood lancet apparatus according to claim 1 or 2, **characterized in that** the cylindrical portion of the cap (102, 202, 302, 402, 502, 602, 702, 802, 902, 1102, 1202, 1302, 1402, 1502, 1602) is arranged between the two arm-like segments (318a, 418a, 518a, 1618a, 318b, 418b, 518b, 1618b) of the cap (102, 202, 302, 402, 502, 602, 702, 802, 902, 1102, 1202, 1302, 1402, 1502, 1602) enclosing the portion (102, 202, 302, 402, 502, 602, 702, 802, 902, 1102, 1202, 1302, 1402, 1502, 1602) in part.

4. A blood lancet apparatus according to claim 3, **characterized in that** the segments (318a, 418a, 518a, 1618a, 318b, 418b, 518b, 1618b) are made uniform.

5. A blood lancet apparatus according to claim 3 or 4, **characterized in that** the segments are arranged in an opposed manner with respect to the cylindrical portion.

6. A blood lancet apparatus according to any one of claims 3 to 5, **characterized in that** the setting wheel (104, 204, 304, 404, 504, 604, 704, 804, 1004, 1304, 1404, 1504) is longer than the segments (318a, 418a, 518a, 1618a, 318b, 418b, 518b, 1618b) or projects at least in part in the longitudinal direction (L) with respect to the segments.

7. A blood lancet apparatus according to claim 6, **characterized in that** a first positioning device (932, 1032, 1132, 1232, 1432, 1532, 1632) and a further positioning device (934, 1034, 1334, 1434, 1534) extend at least in part in the longitudinal direction (L), and the first positioning device (932, 1032, 1132, 1232, 1432, 1532, 1632) consists of at least one pin (932, 1032, 1132, 1232, 1432, 1532, 1632) and the further positioning device (934, 1034, 1334, 1434, 1534) is made undulating, in particular tooth-like, wherein the at least one pin (932, 1032, 1132, 1232, 1432, 1532, 1632) is capable of being deflected at least for a time.

8. A blood lancet apparatus according to claim 7, **characterized in that** the further positioning device (934, 1034, 1334, 1434, 1534) surrounds at least in part the cylindrical portion (626, 726, 826) and has an external set of teeth (934, 1034, 1334, 1434, 1534) and the teeth have tooth-flanks, of which the majority is substantially equal.

9. A blood lancet apparatus according to claim 8, **characterized in that** at least one pin (932, 1032, 1132, 1232, 1432, 1532, 1632) is formed on the setting wheel (104, 204, 304, 404, 504, 604, 704, 804,1004,1304, 1404, 1504).

10. A blood lancet apparatus according to claim 8, **characterized in that** the first positioning device (932, 1032, 1132, 1232, 1432, 1532, 1632) is surrounded at least in part by the setting wheel (104, 204, 304, 404, 504, 604, 704, 804, 1004, 1304, 1404, 1504), the setting wheel has at least in part an internal set of teeth (934, 1034, 1334, 1434, 1534) and the teeth have tooth-flanks, of which the majority is substantially equal.

11. A blood lancet apparatus according to claim 10, **characterized in that** a pin (932, 1032, 1132, 1232, 1432, 1532, 1632) is formed on the - at least in part - cylindrical portion (626, 726, 826), and at least two pins (932, 1032, 1132, 1232, 1432, 1532, 1632) arranged at a distance from each other are preferably formed on the portion.

12. A blood lancet apparatus according to claims 7 to 11, **characterized in that** at least one tooth (936, 1036, 1336, 1436) of the further positioning device (934, 1034, 1334, 1434, 1534) has a flank which is made steeper as compared with the majority of the flanks, wherein the rotational movement of the setting wheel (104, 204, 304, 404, 504, 604, 704, 804, 1004, 1304, 1404, 1504) is restricted by this flank.

13. A blood lancet apparatus according to any one of the preceding claims, **characterized in that** the cap (102, 202, 302, 402, 502, 602, 702, 802, 902, 1102, 1202, 1302, 1402, 1502, 1602) and the setting wheel (104, 204, 304, 404, 504, 604, 704, 804, 1004, 1304, 1404, 1504) have at least in part markings (106a, 206a, 106b, 206b) for determining the exit length of the lancet element.

## Revendications

1. Dispositif de lancette de sang, avec un corps de base (103, 203, 303, 403, 503) pour agencer des composants du dispositif, un dispositif porte-lancette pour maintenir au moins un élément de lancette entourée au moins par sections par le corps de base (103, 203, 303, 403, 503), et un capuchon (102, 202, 302, 402, 502, 602, 702, 802, 902, 1102, 1202, 1302, 1402, 1502, 1602) couplé avec le corps de base pour agencer une molette de réglage (104, 204, 304, 404, 504, 604, 704, 804, 1004, 1304, 1404, 1504) sur un tronçon (626, 726, 826) au moins partiellement cylindrique du capuchon, afin de régler une pluralité de longueurs d'extraction (L1, L2, L3) de l'élément de lancette hors du dispositif de lancette de sang (101, 201, 301, 401, 501) dans lequel la molette de réglage est entourée par des segments (318a, 418a, 518a, 1618a, 318b, 418b, 518b, 1618b) en forme de bras et orientés dans la direction longitudinale (L), de telle sorte qu'un actionnement de la molette de réglage, nécessaire pour le réglage de la longueur d'extraction, peut être effectué uniquement dans les tronçons périphériques sans segments, et deux dispositifs de positionnement (932, 1032, 1132, 1232, 1432, 1532, 1632, 934, 1034, 1334, 1434, 1534) sont prévus, dans lequel l'un des deux dispositifs de positionnement est réalisé contre le capuchon et l'autre dispositif de positionnement est réalisé contre la molette de réglage,
**caractérisé en ce que**
la molette de réglage présente un tronçon fileté intérieur (624, 724, 824, 1524) et le tronçon cylindrique (626, 726, 826) présente un tronçon fileté extérieur (628, 728, 828, 1328, 1528, 1628), dans lequel les tronçons filetés sont reliés fonctionnellement ensemble de telle sorte que la molette de réglage est capable de tourner et est réglable dans la direction longitudinale (L) du corps de base, et
l'autre dispositif de positionnement présente des dents avec des flancs et au moins une dent de l'autre dispositif de positionnement présente un flanc qui est plus pentu que la pluralité des flancs.

2. Dispositif de lancette de sang selon la revendication 1, caractérisé en ce le capuchon (102, 202, 302, 402, 502, 602, 702, 802, 902, 1102, 1202, 1302, 1402, 1502, 1602) et/ou le corps de base (103, 203, 303, 403, 503) présentent une section transversale qui dans une première direction transversale (B) est supérieure au diamètre de la molette de réglage (104, 204, 304, 404, 504, 604, 704, 804, 1004, 1304, 1404, 1504), et dans une seconde direction transversale (T) est inférieure à la section transversale dans la première direction transversale (B).

3. Dispositif de lancette de sang selon la revendication 1 ou 2, **caractérisé en ce que** le tronçon cylindrique du capuchon (102, 202, 302, 402, 502, 602, 702, 802, 902, 1102, 1202, 1302, 1402, 1502, 1602) est agencé entre les deux segments en forme de bras (318a, 418a, 518a, 1618a, 318b, 418b, 518b, 1618b) du capuchon (102, 202, 302, 402, 502, 602, 702, 802, 902, 1102, 1202, 1302, 1402, 1502, 1602), lesquels entourent partiellement le tronçon (102, 202, 302, 402, 502, 602, 702, 802, 902, 1102, 1202, 1302, 1402, 1502, 1602).

4. Dispositif de lancette de sang selon la revendication 3, **caractérisé en ce que** les segments (318a, 418a, 518a, 1618a, 318b, 418b, 518b, 1618b) sont réalisés avec la même forme.

5. Dispositif de lancette de sang selon les revendications 3 ou 4, **caractérisé en ce que** les segments sont agencés face à face par rapport au tronçon cylindrique.

6. Dispositif de lancette de sang selon l'une quelconque des revendications 3 à 5, **caractérisé en ce que** la molette de réglage (104, 204, 304, 404, 504, 604, 704, 804, 1004, 1304, 1404, 1504) est plus longue que les segments (318a, 418a, 518a, 1618a, 318b, 418b, 518b, 1618b) ou est au moins partiellement en saillie par rapport aux segments dans la direction longitudinale (L).

7. Dispositif de lancette de sang selon la revendication 6, **caractérisé en ce qu'**un premier dispositif de positionnement (932, 1032, 1132, 1232, 1432, 1532, 1632) et un autre dispositif de positionnement (934, 1034, 1334, 1434, 1534) s'étendent au moins partiellement dans la direction longitudinale (L), et le premier dispositif de positionnement (932, 1032, 1132, 1232, 1432, 1532, 1632) comporte au moins une broche (932, 1032, 1132, 1232, 1432, 1532, 1632) et l'autre dispositif de positionnement (934, 1034, 1334, 1434, 1534) est réalisé avec une forme ondulée, en particulier une forme dentée, dans lequel la au moins une broche (932, 1032, 1132, 1232, 1432, 1532, 1632) peut être déviée au moins temporairement.

8. Dispositif de lancette de sang selon la revendication 7, **caractérisé en ce que** l'autre dispositif de positionnement (934, 1034, 1334, 1434, 1534) entoure au moins partiellement le tronçon cylindrique (626, 726, 826) et présente une denture extérieure (934, 1034, 1334, 1434, 1534) et les dents présentent des flancs dont la pluralité est réalisée de manière sensiblement identique.

9. Dispositif de lancette de sang selon la revendication 8, **caractérisé en ce qu'**au moins une broche (932, 1032, 1132, 1232, 1432, 1532, 1632) est réalisé contre la molette de réglage (104, 204, 304, 404, 504, 604, 704, 804, 1004, 1304, 1404, 1504).

10. Dispositif de lancette de sang selon la revendication 8, **caractérisé en ce que** le premier dispositif de positionnement (932, 1032, 1132, 1232, 1432, 1532, 1632) est entouré au moins partiellement par la molette de réglage (104, 204, 304, 404, 504, 604, 704, 804, 1004, 1304, 1404, 1504), la molette de réglage présente au moins partiellement une denture intérieure (934, 1034, 1334, 1434, 1534) et les dents présentent des flancs dont la pluralité est réalisée de manière sensiblement identique.

11. Dispositif de lancette de sang selon la revendication 10, **caractérisé en ce qu'**une broche (932, 1032, 1132, 1232, 1432, 1532, 1632) est formée contre le tronçon (626, 726, 826) au moins partiellement cylindrique, de préférence deux broches (932, 1032, 1132, 1232, 1432, 1532, 1632), à distance l'une de l'autre, sont formées contre le tronçon.

12. Dispositif de lancette de sang selon les revendications 7 à 11, **caractérisé en ce qu'**au moins une dent (936, 1036, 1336, 1436) de l'autre dispositif de positionnement (934, 1034, 1334, 1434, 1534) présente un flanc plus pentu que la pluralité des flancs, dans lequel le mouvement de rotation de la molette de réglage (104, 204, 304, 404, 504. 604, 704, 804, 1004, 1304, 1404, 1504) est limité par l'intermédiaire de ce flanc.

13. Dispositif de lancette de sang selon l'une quelconque des revendications précédentes, **caractérisé en ce que** le capuchon (102, 202, 302, 402, 502, 602, 702, 802, 902, 1102, 1202, 1302, 1402, 1502, 1602) et la molette de réglage (104, 204, 304, 404, 504, 604, 704, 804, 1004, 1304, 1404, 1504) présentent au moins en partie des marquages (106a, 206a, 106b, 206b) pour déterminer la longueur d'extraction de l'élément de lancette.
